(19) Europäisches Patentamt
European Patent Office
Office européen des brevets

(11) Numéro de publication: **0 093 050**
**B1**

(12) **FASCICULE DE BREVET EUROPÉEN**

(45) Date de publication du fascicule du brevet: **15.01.86**

(51) Int. Cl.[4]: **C 07 D 519/00,** A 61 K 31/495 // (C07D519/00, 495:00, 471:00)

(21) Numéro de dépôt: **83400794.0**

(22) Date de dépôt: **21.04.83**

(54) **Nouveaux dérivés du dithiinno (1,4)(2,3-c)pyrrole, leur préparation et les médicaments qui les contiennent.**

(30) Priorité: **22.04.82 FR 8206944**

(43) Date de publication de la demande: **02.11.83 Bulletin 83/44**

(45) Mention de la délivrance du brevet: **15.01.86 Bulletin 86/3**

(84) Etats contractants désignés: **AT BE CH DE FR GB IT LI LU NL SE**

(56) Documents cité: **US-A-4 220 646**

**Le dossier contient des informations techniques présentées postérieurement au dépôt de la demande et ne figurant pas dans le présent fascicule.**

(73) Titulaire: **RHONE- POULENC SANTE, Les Miroirs 18 Avenue d'Alsace, F-92400 Courbevoie Cedex (FR)**

(72) Inventeur: **Farge, Daniel, 30, rue des Pins Sylvestres, F-94320 Thiais (FR)**
Inventeur: **Leger, André, 97, rue des Morillons, F-75015 Paris (FR)**
Inventeur: **Ponsinet, Gérard, 7, rue de Grand Champ, F-94370 Sucy- en- Brie (FR)**

(74) Mandataire: **Le Goff, Yves, RHONE- POULENC RECHERCHES Brevets Pharma 25, Quai Paul Doumer, F-92408 Courbevoie (FR)**

**EP 0 093 050 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne de nouveaux dérivés du dithiinno [1,4][2,3-c] pyrrole de formule générale:

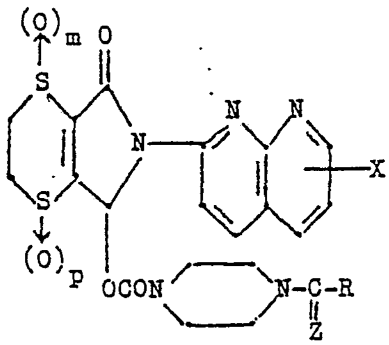

(I)

leur préparation et les médicaments que les contiennnent. Dans la formule générale (I), X représente un atome d'hydrogène ou d'halogène ou bien un radical alcoyle ou alcoyloxy contenant 1 à 4 atomes de carbone, cyano ou nitro. R représente un atome d'hydrogène ou un radical alcoyle contenant 1 à 4 atomes de carbone non substitué ou substitué par 1, 2 ou 3 atomes d'halogène, ou un radical alcényle contenant 2 à 4 atomes de carbone, alcynyle contenant 2 à 4 atomes de carbone, ou alcoyloxy contenant 1 à 4 atomes de carbone, ou bien phényle non substitué par 1, 2 ou 3 atomes d'halogène, un radical alcoyle contenant 1 à 4 atomes de carbone, alcoyloxy contenant 1 à 4 atomes de carbone, nitro ou trifluorométhyle ou bien phényalcoyle dont la partie alcoyle contient 1 à 4 atomes de carbone ou phénylalcynyle dont la partie alcynyle contient 2 à 4 atomes de carbone et dont les noyaux phényle sont éventuellement substitués comme indiqué perécédemment, Z représente un atome d'oxygène ou de soufre et m et p sont des nombres entiers égaux à 0 ou 1 étant entendu que m est égal à 0 lorsque p est égal à 1 et que m est égal à 1 lorsque p est égal à 0, et que tous les radicaux alcoyle, alcényle ou alcynyle sont en chaînes droites ou ramifiées.

Il est entendu de plus que la présente invention concerne les produits de formule générale (I) sous les formes cis et trans pures et en mélanges.

Selon l'invention, les produits de formule générale (I) peuvent être obtenus par oxydation du produit de formule générale:

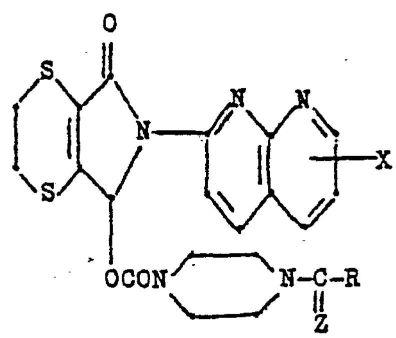

(II)

dans laquelle R, Z et X sont définis comme précédemment, suivie d'une séparation des produits obtenus.

L'oxydation peut être réalisée en employant un équivalent d'un agent couramment utilisé pour passer d'un sulfure à un sulfoxyde, en opérant dans un solvant approprié. Par exemple, on peut employer l'eau oxygénée dans l'acétone ou l'acide acétique, un périodate alcanin dans un alcool ou l'acétonitrile, un peroxyacide carboxylique (acide peracétique, perbenzoïque, m-chloroperbenzoïque, p-nitroperbenzoïque ou perphtalique) dans un éther (dioxanne, tétrahydrofuranne, oxyde de diéthyle), un solvant chloré (dichlorométhane, dichloroéthane), l'acide acétique ou un mélange de ces solvants. La réaction s'effectue généralement à une température comprise entre −10 et +20°C.

Il est particulièrement avantageux d'opérer dans un mélange d'acide acétique et de dichlorométhane en présence d'acide m-chloroperbenzoïque, à une température comprise entre −10 et 0°C.

La séparation des différents produits d'oxydation peut être effectuée par tout moyen physique ou chimique habituel à la portée de l'homme de métier. Il est particulièrement avantageux d'effectuer la séparation par chromatographie.

Les produits de formule générale (II) peuvent être préparés comme décrit dans le brevet américain 4 220 646. Sont particulièrement intéressants les produits de formule générale (I) dans laquelle X représente un atome d'halogène, Z représente un atome d'oxygène et R représente un radical alcoyle contenant 1 à 4 atomes de carbone et m et p sont définis comme précédemment.

D'un intérêt particulier sont les produits suivants: -(chloro-7 naphtyridin-[1,8] yl -2)-6 (propionyl-4 pipé-razinyl-1) carbonyloxy-5 oxo-7 tétrahydro-2, 3, 6, 7 5H-dithiinno [1,4] [2,3-c] pyrrole - oxyde-1 -(chloro-7 naphtyridin-[1,8] yl -2)-6 (propionyl-4 pipérazinyl-1) carbonylox-y-5 oxo-7 tétrahydro- 2, 3, 6, 7 5H-dithiinno [1,4] [2,3-c] pyrrole-oxyde-4

Les produits de formule générale (II) ont une action tranquillisante, anticonvulsivante et hypnogene. Il a maintenant été trouvé que les nouveaux produits selon l'invention, c'est-à-dire les monosulfoxydes correspondants, possèdent une activité tranquillisante et anticonvulsivante voisine de celle des produits de formule générale (II) mais présentent des propriétés "hypnogènes" plus faibles susceptibles de les rendre plus aptes au traitement de certaines maladies (états anxieux, épilepsie,...).

L'activité tranquillisante des produits selon l'invention peut être mise en évidence chez l'animal (souris) à des doses comprises entre 1 et 10 mg/kg par voie orale dans le test des convulsions induites par le pentétrazol selon une technique voisine de celle de EVERETT et RICHARDS [J. Pharmacol. Exp.Ther. 81, 402,(1964)].

Chez la souris, par voie orale, dans le test de potentialisation d'une faible dose de chlorpromazine mesurée dans le test de Righting Reflex [selon ZBINDEN et RANDALL, Advances in Pharmacology 5, 213-291 (1967)] (test permettant de prévoir les effets "hypnogènes" d'un produit), les produits ne se sont montrés actifs qu'à des doses supérieures à 30 mg/kg.

De plus, les produits selon l'invention présentent une faible toxicité. Chez la souris, la toxicité aiguë (exprimée par sa $DL_{50}$) est généralement comprise entre 300 et 900 mg/kg ou même supérieure à 900 mg/kg.

L'exemple suivant, donné à titre non limitatif, montre comment l'invention peut être mise en pratique.

**EXEMPLE**

A une solution refroidie à -10°C de 5 g de (chloro-7 naphtyridin [1,8] yl-2)-6 oxo-7 (propionyl-4 pipérazinyl-1) carbonyloxy-5 tétrahydro-2, 3, 6,-7 5H-dithinno [1,4][2,3-c] pyrrole dans 100 cm3 de dichlorométhane, on ajoute une solution de 2,32 g d'acide chloro-3 perbenzoique à 80 % dans 65 cm3 de dichlorométhane. L'addition est effectuée goutte à goutte en 30 minutes, de façon à maintenir la température entre -10 et -5°C. On agite encore une heure à cette température, puis verse la solution dans 400 cm3 d'oxyde de diéthyle refroidi à 0°C. Le précipité qui se forme est séparé par filtration, lavé avec 60 cm3 d'oxyde de diéthyle et séché à l'air.

Le produit obtenu est chromatographié sur 100 g de gel de silice (0,04-0,06 mm) contenus dans une colonne de 4 cm de diamètre. On élue par un mélange de 2910 cm3 de chloroforme et 90 cm3 de méthanol en recueillant des fractions de 70 cm3.

Les fractions 5 à 19 sont réunies et concentrées à sec sous pression réduite (20 mm de mercure; 2,7 kPa) à 40°C. Le résidu est trituré dans 30 cm3 d'oxyde de diispropyle. Le solide obtenu est filtré, lavé avec 10 cm3 d'oxyde de diisopropyle et séché sous pression réduite (0,2 mm de mercure; 0,027 kPa) à 40°C. On obtient ainsi 4,3 g de (chloro -7. naphtyridin [1,8] yl-2)-6 (propionyl-4 pipérazinyl-1) carbonyloxy-5 oxo-7 tétrahydro-2, 3, 6, 7 5H-dithiinno [1,4][2,3-c] pyrrole-oxyde-1, sous forme d'un mélange possible des formes cis et trans fondant avec décomposition à 206° C.

On réunit les fractions 27 à 39, concentre à sec sous pression réduite (20 mm de mercule; 2,7 kPa) à 40°C. Le résidu est trituré dans 30 cm3 d'oxyde de diisopropyle. Le solide obtenu est filtré, lavé avec 10 cm3 d'oxyde de diisopropyle et séché sous pression réduite (0,2 mm de mercure; 0,027 kPa) à 40°C. On obtient ainsi 0,5 g de (chloro-7 naphtyridin [1,8] yl-2)-6 (propionyl-4 pipérazinyl-1) carbonyloxy-5 oxo-7 tétrahydro-2, 3, 6, 7 5H-dithiinno [1,4][2,3-c] pyrrole-oxyde-4 correspondant à la forme cis ou trans et fondant avec décomposition à 220°C.

La présente invention concerne également les médicaments constitués par un produit de formule générale (I), à l'état pur ou sous forme d'une composition dans laquelle il est associé à tout autre produit pharmaceutiquement compatible, pouvant être inerte ou physiclogiquement actif. Les médicaments selon l'invention peuvent être utilisés par voie orale, parentérale ou rectale.

Comme compositions solides pour administration orale peuvent être utilisée des comprimés, pilules, poudres (notamment dans des capsules de gélatine ou des cachets) ou granulés. Dans ces compositions, le produit actif selon l'invention est mélangé à un ou plusieurs diluants inertes, tels que amidon, cellulose, saccharose, lactose ou silice. Ces compositions peuvent également comprendre des substances autres que les diluants, par exemple un ou plusieurs lubrifiants tel que la stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent également comprendre des substances autres que les diluants, par exemple des produits mouillants, édulcorants, épaississants, aromatisants ou stabilisants.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le produit actif des excipients tels que le beurre de cacao, des glycérides semi-synthétiques ou des

polyéthylèneglycols.

En thérapeutique humaine, les produits selon l'invention sont particulièrement utiles dans le traitement de certaines maladies se manifestant par des états anxieux ou épileptiformes. Les doses dépendent de l'effet rocherché et de la durée du traitement; elles sont généralement comprises entre 1 et 100 mg par jour par voie orale peur un adulte en une ou plusieurs prises.

D'une façon générale, le médecin déterminera la posologie qu'il estime la plus appropriée en fonction de l'âge, du poids et de tous les autres facteurs propres au sujet à traiter.

L'exemple suivant, donné à titre non limitatif, illustre des compositions selon l'invention.

**EXEMPLE** On prépare, selon la technique habituelle, des comprimés dosés à 10 mg de produit actif ayant la composition suivante:

- (chloro-7 naphtyridin-[1,8] yl-2)-6 (propionyl-4 pipérazinyl-1) carbonyloxy-5 oxo-7 tétrahydro-2,3,6,7 5H-dithiinno [1,4][2,3-c] pyrrole -oxyde-1... 10 mg
- amidon... 60 mg
- lactose... 50 mg
- stéarate de magnésium... 2 mg

**Revendications**

Pour les Etats Coutractants BE CH DE FR GB IT U LU NL SE.

1 - Un dérivé du dithiinno [1,4][2,3-c] pyrrole caractérisé en ce qu'il répond à la formule générale:

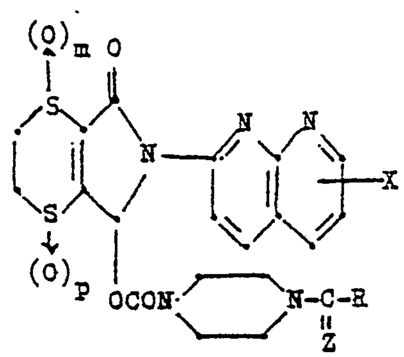

(I)

dans laquelle X représente un atome d'hydrogène ou d'halogène ou bien un radical alcoyle ou alcoyloxy contenant 1 à 4 atomes de carbone, cyano ou nitro, R représente un atome d'hydrogène ou un radical alcoyle contenant 1 à 4 atomes de carbone [non substitué ou substitué par 1, 2 ou 3 atomes d'halogène, ou un radical alcényle contenant 2 à 4 atomes de carbone, alcynyle contenant 2 à 4 atomes de carbone, alcoyloxy contenant 1 à 4 atomes de carbone], phényle (non substitué ou substitué par 1, 2 ou 3 atomes d'halogène, un radical alcoyle contenant 1 à 4 atomes de carbone, alcoyloxy contenant 1 à 4 atomes de carbone, nitro ou trifluorométhyle), phénylalcoyle dont la partie alcoyle contient 1 à 4 atomes de carbone ou phénylalcynyle dont la partie alcynyle contient 2 à 4 atomes de carbone et dont les noyaux phényle sont éventuellement substitués comme indiqué précédemment, Z représente un atome d'oxygène ou de soufre et m et p sont des nombres entiers égaux à 0 ou 1 étant entendu que m est égal à 0 lorsque p est égal à 1 et m est égal à 1 lorsque p est égal à 0.

2 - Procédé de préparation d'un produit selon la revendication caractérisé en ce que l'on oxyde un produit de formule générale:

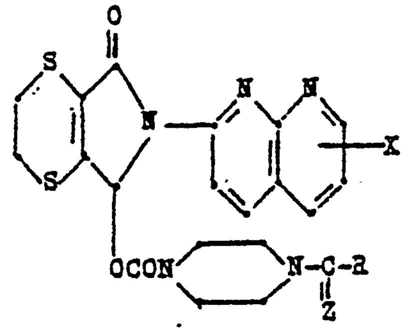

(II)

puis sépare et isole les produits obtenus.

3 - Procédé selon la revendication 2, caractérisé en ce que l'on oxyde le produit de formule générale (II) au moyen d'un agent d'oxydation choisi dans le groupe constitué par l'eau oxygénée, les périodates alcalins et les

peroxyacides carboxyliques et que l'on utilise cet agent à raison d'environ un équivalent par mole de produit à oxyder.

4 - Un médicament caractérisé en ce qu'il contient un produit selon la revendication 1 en association avec un ou plusieurs diluants ou adjuvants compatibles et pharmaceutiquement acceptables.

**Revendications**

Porur l'etat Contractant: AT 1 - Procédé de préparation d'un dérivé du dithiinno [1,4] [2,3-c] pyrrole de formule générale.:

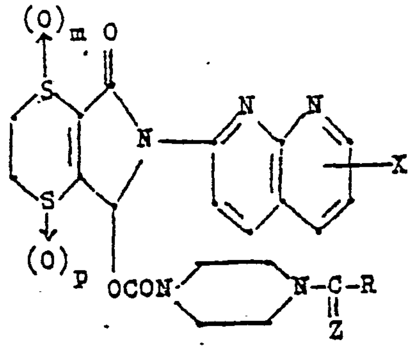

(I)

dans laquelle X représente un atome d'hydrogène ou d'halogène ou bien un radical alcoyle ou alcoyloxy contenant 1 à 4 atomes de carbone, cyano ou nitro, R représente un atome d'hydrogène ou un radical alcoyle contenant 1 à 4 atomes de carbone [non substitué ou substitué par 1, 2 ou 3 atomes d'halogène, ou un radical alcényle contenant 2 à 4 atomes de carbone, alcynyle contenant 2 à 4 atomes de carbone, alcoyloxy contenant 1 à 4 atomes de carbone], phényle (non substitué ou substitué par 1, 2 ou 3 atomes d'halogène, un radical alcoyle contenant 1 à 4 atomes de carbone, alcoyloxy contenant 1 à 4 atomes de carbone, nitro ou trifluorométhyle), phénylalcoyle dont la partie alcoyle contient 1 à 4 atomes de carbone ou phénylalcynyle dont la partie alcynyle contient 2 à 4 atomes de carbone et dont les noyaux phényle sont éventuellement substitués comme indiqué précédemment, Z représente un atome d'oxygène ou de soufre et m et p sont des nombres entiers égaux à 0 ou 1 étant entendu que m est égal à 0 lorsque p est égal à 1 et m est égal à 1 lorsque p est égal à 0, caractérisé en ce que l'on oxyde un produit de formule générale:

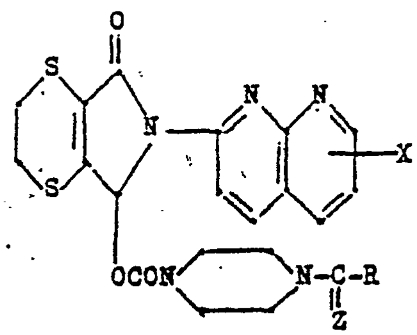

(II)

puis sépare et isole les produits obtenus.

2 - Procédé selon la revendication 1 caractérisé en ce que l'oxydation est effectuée au moyen de tout agent d'oxydation connu pour oxyder un sulfure organique en sulfoxyde correspondant.

3 - Procédé selon la revendication 2 caractérisé en ce que l'on oxyde le produit de formule générale (II) au moyen d'un agent d'oxydation choisi dans le groupe constitue par l'eau oxygénée, les périodates alcalins et les peroxyacides carboxyliques et que l'on utilise cet agent à raison d'environ un équivalent par Iole de produit à oxyder.

**Claims**

for the contracting state: AT

1 - Process for preparing a [1,4]dithiino[2,3-c]-pyrrole derivative of general formula:

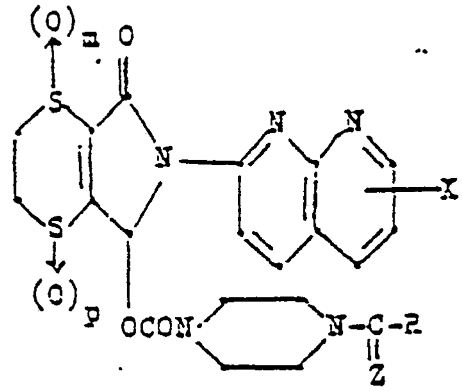

(I)

in which X denotes a hydrogen or halogen atom or alternatively an alkyl or alkyloxy radical containing 1 to 4 carbon atoms, cyano or nitro, R denotes a hydrogen atom or an alkyl radical containing 1 to 4 carbon atoms [unsubstituted or substituted with 1, 2 or 3 halogen atoms, or an alkenyl radical containing 2 to 4 carbon atoms, an alkynyl radical containing 2 to 4 carbon atoms, or an alkyloxy radical containing 1 to 4 carbon atoms], phenyl (unsubstituted or substituted with 1, 2 or 3 halogen atoms, an alkyl radical containing 1 to 4 carbon atoms, or an alkyloxy radical containing 1 to 4 carbon atoms, nitro or trifluoromethyl), phenylalkyl in which the alkyl portion contains 1 to 4 carbon atoms or phenylalkynyl in which the alkynyl portion contains 2 to 4 carbon atoms and in which the phenyl rings are optionally substituted as mentioned above, Z denotes an oxygen or sulphur atom and m and p are integers equal to 0 or 1, on the understanding that m equals 0 when p equals 1 and m equals 1 when p equals 0, characterised in that a product of general formula:

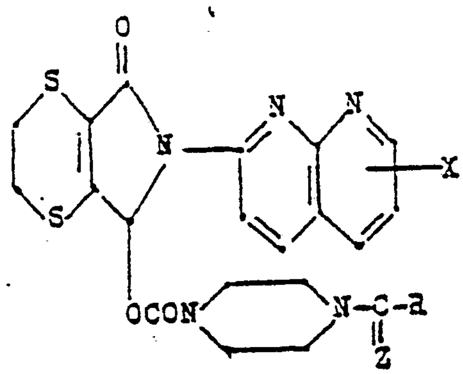

(II)

is oxidised, and the products obtained are then separated and isolated.

2 - Process according to Claim 1, characterised in that the oxidation is performed by means of any oxidising agent known for oxidising an organic sulphide to the corresponding sulphoxide.

3 - Process according to Claim 2, characterised in that the product of general formula (II) is oxidised by means of an oxidising agent chosen from the group consisting of hydrogen peroxide, alkali metal periodates and percarboxylic acids, and that this agent is used in the proportion of approximately one equivalent per mole of product to be oxidised.

**Claims**

for the contracting states BE, CH, DE, FR, GB, IT, LI, LU, NL, SE

1 - A [1,4]dithiino[2,3-c]pyrrole derivative, characterized in that it corresponds to the general formula:

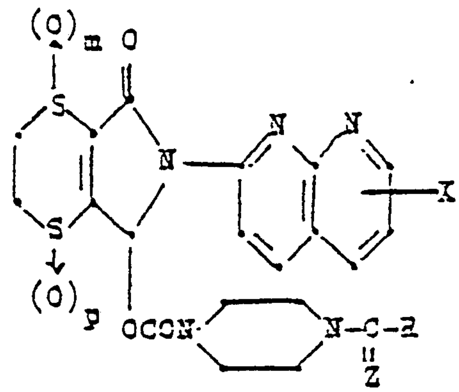

(I)

in which X denotes a hydrogen or halogen atom or alternatively an alkyl or alkyloxy radical containing 1 to 4 carbon atoms, cyano or nitro, R denotes a hydrogen atom or an alkyl radical containing 1 to 4 carbon atoms [unsubstituted or substituted with 1, 2 or 3 halogen atoms, or an alkenyl radical containing 2 to 4 carbon atoms, an alkynyl radical containing 2 to 4 carbon atoms, or an alkyloxy radical containing 1 to 4 carbon atoms], phenyl (unsubstituted or substituted with 1, 2 or 3 halogen atoms, an alkyl radical containing 1 to 4 carbon atoms, or an alkyloxy radical containing 1 to 4 carbon atoms, nitro or trifluoromethyl), phenylalkyl in which the alkyl portion contains 1 to 4 carbon atoms or phenylalkynyl in which the alkynyl portion contains 2 to 4 carbon atoms and in which the phenyl rings are optionally substituted as mentioned above, Z denotes an oxygen or sulphur atom and m and p are integers equal to 0 or 1, on the understanding that m equals 0 when p equals 1 and m equals 1 when p equals 0.

2 - Process for preparing the products according to Claim 1, characterised in that a product of general formula:

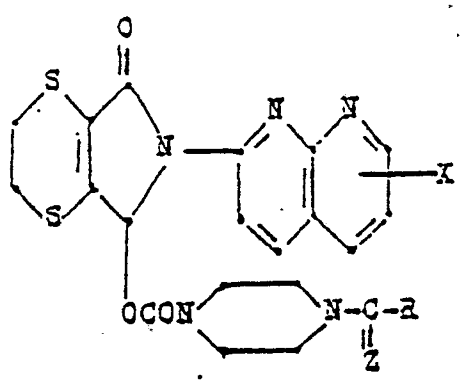

(II)

is oxidised, and the products obtained are then separated and isolated.

3 - Process according to Claim 2, characterised in that the product of general formula (II) is oxidised by means of an oxidising agent chosen from the group consisting of hydrogen peroxide, alkali metal periodates and percarboxylic acids, and that this agent is used in the proportion of approximately one equivalent per mole of product to be oxidised.

4 - A drug, characterised in that it contains a product according to Claim 1 in combination with one or more diluents or adjuvants which are compatible and pharmaceutically acceptable.

**Patentansprüche:**

für den Vertragsstaat AT

1. Verfahren zur Herstellung eines Derivats des [1,4]-Dithiinno-[2,3-c]-pyrrols der allgemeinen Formel:

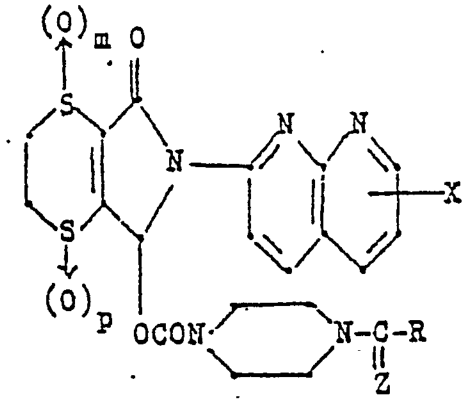

(I),

in welcher X ein Wasserstoff- oder Halogenatom oder einen Alkyloder Alkyloxyrest mit 1 bis 4 Kohlenstoffatomen, einen Cyano- oder Nitrorest darstellt, R für ein Wasserstoffatom oder einen Alkylrest mit 1 bis 4 Kohlenstoffatomen [gegebenenfalls substituiert durch 1, 2 oder 3 Halogenatome oder einen Alkenylrest mit 2 bis 4 Kohlenstoffatomen, einen Alkynylrest mit 2 bis 4 Kohlenstoffatomen, einen Alkyloxyrest mit 1 bis 4 Kohlenstoffatomen], einen Phenylrest mit (gegebenenfalls substituiert durch 1, 2 oder 3 Halogenatome, einen Alkylrest mit 1 bis 4 Kohlenstoffatomen, einen Alkyloxyrest mit 1 bis 4 Kohlenstoffatomen, einen Nitro- oder Trifluormethylrest), einen Phenylalkylrest, dessen Alkylteil 1 bis 4 Kohlenstoffatome enthält, oder einen Phenylalkynylrest, dessen Alkynylteil 2 bis 4 Kohlenstoffatome enthält und deren Phenylkerne gegebenenfalls wie oben angegeben substituiert sind, steht, Z ein Sauerstoff- oder Schwefelatom darstellt und m und p ganze Zahlen gleich 0 oder 1 sind, wobei selbstverständlich m gleich 0 ist, wenn p gleich 1 ist, und m gleich 1 ist, wenn p gleich 0 ist, dadurch gekennzeichnet, daß man eine Verbindung der allgemeinen Formel:

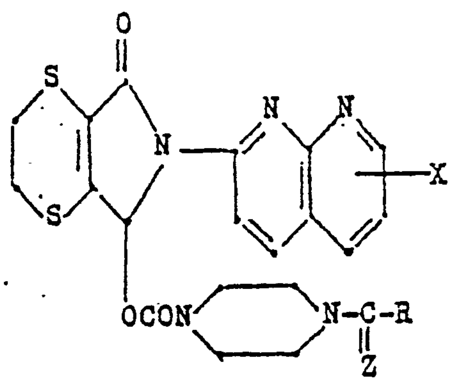

(II)

oxidiert und dann die erhaltenen Verbindungen trennt und isoliert.

2. Verfahren nach Anspruch 1, dadurch gekennzeichnet, daß die Oxidation mittels irgendeines bekannten Oxidationsmittels für die Oxidation eines organischen Sulfids zum entsprechenden Sulfoxid ausgeführt wird.

3. Verfahren nach Anspruch 2, dadurch gekennzeichnet, daß man die Verbindung der allgemeinen Formel (II) mittels eines Oxidationsmittels, ausgewählt aus der Gruppe, bestehend aus Wasserstoffperoxid, den Alkaliperjodaten und den Peroxycarbonsäuren, oxidiert und daß man dieses Mittel in einer Menge von etwa einem Äquivalent pro Mol zu oxidierender Verbindung einsetzt.

**Patentansprüche**

für die Vertragsstaaten
BE, CH, DE, FR, GB, IT, LI, LU, NL, SE

1. Ein Derivat des Dithiinno [1,4][2,3-c] pyrrols, dadurch gekennzeichnet, daß es der allgemeinen Formel I

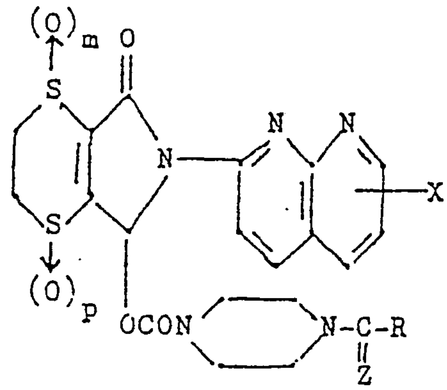

(I)

entspricht, worin X ein Wasserstoff- oder Halogenatom oder auch einen Alkyl- oder Alkoxyrest mit 1 bis 4 Kohlenstoffatomen, Cyano oder Nitro bedeutet, R ein Wasserstoffatom oder einen Alkylrest mit 1 bis 4 Kohlenstoffatomen [ nicht substituiert oder substituirt durch 1, 2 oder 3 Halogenatome oder einen Alkenylrest mit 2 bis 4 Kohlenstoffatomen, Alkinylrest mit 2 bis 4 Kohlenstoffatomen, Alkoxyrest mit 1 bis 4 Kohlenstoffatomen], Phenyl (nicht substituiert oder substituiert durch 1, 2 oder 3 Halogenatome, einen Alkylrest mit 1 bis 4 Kohlenstoffatomen, Alkyloxy mit 1 bis 4 Kohlenstoffatomen Nitro oder Trifluormethyl), Phenylalkyl, dessen Alkylteil 1 bis 4 Kohlenstoffatome enthält oder Phenylalkinyl, dessen Alkinylteil 2 bis 4 Kohlenstoffatome enthält, wobei die Phenylringe gegebenenfalls wie vorstehend angegeben substituiert sind, bedeutet, Z ein Sauerstoff- oder Schwefelatom bedeutet und m und p ganze Zahlen gleich 0 oder 1 sind, wobei m gleich 0 ist, wenn p = 1 und m = 1, wenn p = 0.

2. Verfahren zur Herstellung eines Produkts gemäß Anspruch 1, dadurch gekennzeichnet, daß man ein Produkt der allgemeinen formel II

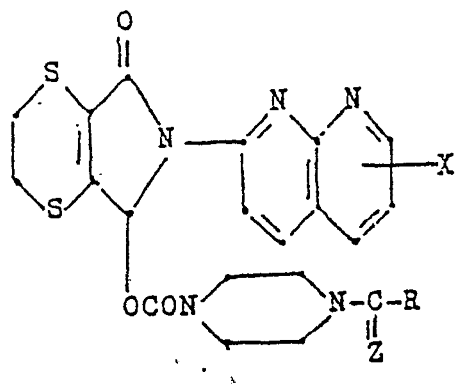

(II)

oxidiert und dann die erhaltenen Produkte abtrennt und isoliert.

3. Verfahren gemäß Anspruch 2, dadurch gekennzeichnet, daß man das Produkt der allgemeinen Formel II mittels eines Oxidationsmittels, ausgewählt aus der Gruppe bestehend aus Wasserstoffperoxid, den Alkaliperiodaten und den carboxylischen Peroxysäuren oxidiert, und daß man dieses Mittel in einer Menge von etwa einem Äquivalent pro Mol zu oxidierendes Produkt verwendet.

4. Arzneimittel, dadurch gekennzeichnet, daß es ein Produkt gemäß Anspruch 1 in Assoziation mit einem oder mehreren verträglichen und pharmazeutisch annehmbaren Verdünnungs- oder Hilfsmitteln enthält.